# EUROPÄISCHE PATENTSCHRIFT

(11) **EP 3 254 474 B1**
(45) Veröffentlichungstag und Bekanntmachung des Hinweises auf die Patenterteilung: **02.10.2019**
(21) Anmeldenummer: 16701930.6
(22) Anmeldetag: 27.01.2016
(51) Int. Cl.: H04R 17/00, A61N 1/05, H04R 25/00, A61N 1/36

(54) **ELEKTROAKUSTISCHES IMPLANTAT**
ELECTRO-ACOUSTIC IMPLANT
IMPLANT ÉLECTROACOUSTIQUE

(30) Priorität: 02.02.2015 DE 102015101482
(43) Veröffentlichungstag der Anmeldung: 13.12.2017
(73) Patentinhaber: Vibrosonic GmbH, 68167 Mannheim (DE)
(72) Erfinder: SCHÄCHTELE, Jonathan, 68159 Mannheim (DE); KALTENBACHER, Dominik, 68163 Mannheim (DE)
(74) Vertreter: Pfenning, Meinig & Partner mbB
(86) Internationale Anmeldenummer: PCT/EP2016/051713
(87) Internationale Veröffentlichungsnummer: WO 2016/124465

(56) Entgegenhaltungen:
- WO-A1-2016/048936
- US-A1- 2004 133 250
- US-A1- 2008 064 918
- US-A1- 2012 053 393
- US-A1- 2012 136 197
- US-B1- 6 190 305

## Beschreibung

Die Erfindung betrifft ein elektroakustisches Implantat mit einem länglichen Elektrodenträger, der eine Mehrzahl an Stimulationselektroden aufweist, sowie mit einem flächigen Schallwandler, der durch Anlegen einer Spannung zumindest bereichsweise zum Schwingen anregbar ist.

Patienten mit an Taubheit grenzender Schwerhörigkeit können heute erfolgreich mit Cochlea-Implantaten (CIs) versorgt werden. Ca. 20 % der für ein CI indizierten Patienten besitzen bei Frequenzen < 1 kHz ausgeprägtes Resthörvermögen, das der Hörfähigkeit bei moderater bis mittlerer Schwerhörigkeit entspricht. Bei einer regulären Versorgung mit einem CI besteht für den Patienten die Gefahr, dieses Resthörvermögen aufgrund der Invasivität der Elektrodenimplantation zu verlieren.

Hörsysteme zur Versorgung hochgradig schwerhöriger Patienten mit Resthörvermögen basieren auf der elektroakustischen Stimulation der Hörorgane. Es ist bekannt, eine Cochlea-Implantatelektrode in die Gehörschnecke einzuführen, um die Hörsinnzellen für hohe Frequenzen anzuregen. Zur akustischen Stimulation der tiefen Frequenzen kann dabei im Gehörgang ein herkömmliches Hörgerät getragen werden. Dabei werden verstärkte Schallwellen von einem Lautsprecher an die Luftsäule im Gehörgang abgegeben. Über Trommelfell und Gehörknöchelchen übertragen sich die verstärkten Schwingungen schließlich auf die Gehörschnecke.

Die US 2004/0133250 A1 beschreibt eine implantierbare Hörhilfe für Personen mit Hörverlust oder Tinnitus. Die Hörhilfe weist mehrere Strukturen zur Stimulation des Ohres mittels mehrerer Signalübertragern auf.

Die US 2008/0064918 A1 beschreibt ein System zur Kommunikation mit dem Innenohr, in dem ein akustischer Schallwandler elektrische Energie in mechanische Energie umwandelt. Ein Innenohrkatheter hat ein distales Ende in schwingender Kommunikation mit der Flüssigkeit des Innenohrs und ein proximales Ende in schwingender Kommunikation mit dem akustischen Schallwandler.

Die US 2012/0053393 A1 beschreibt einen Schallübertrager zum Erzeugen von Schwallschwingungen, der in ein Ohr implantiert werden kann und als implantierbare Hörhilfe verwendet werden kann. Der Schallwandler weist mindestens eine Trägerschicht und zumindest eine piezoelektrische Schicht auf, die durch Anlegen einer Spannung zum Schwingen gebracht werden können.

Die US 6,190,305 B1 beschreibt einen piezoelektrischen "floating mass"-Schallwandler mit dünner Membran.

Problematisch im Stand der Technik ist, dass die Implantation normalerweise sehr aufwendig und kompliziert ist. Die meisten Lösungen erfordern ein sichtbar am Außenohr getragenes Hörgerät. Die Tonqualität ist häufig aufgrund von Rückkopplungen und Verzerrungen über den Gehörgang eingeschränkt und viele Geräte funktionieren nur mit intaktem Mittelohr.

Aufgabe der vorliegenden Erfindung ist es, die Nachteile des Standes der Technik zu überwinden.

Diese Aufgabe wird gelöst durch das elektroakustische Implantat nach Anspruch 1. Die abhängigen Ansprüche geben vorteilhafte Weiterbildungen des elektroakustischen Implantats gemäß Anspruch 1 an.

Erfindungsgemäß wird ein elektroakustisches Implantat angegeben, also ein Hörgerät, das unter anderem Hörorgane eines Patienten elektrisch stimulieren kann, um einen Höreindruck zu erzeugen.

Erfindungsgemäß weist das elektroakustische Implantat einen länglichen Elektrodenträger mit einer Mehrzahl an Stimulationselektroden auf. Der längliche Elektrodenträger ist so ausgestaltet, dass er in eine Hörschnecke eines Patienten einführbar ist. Durch die Mehrzahl an Stimulationselektroden sind Hörsinneszellen in der Gehörschnecke elektroakustisch stimulierbar.

Das erfindungsgemäße elektroakustische Implantat weist außerdem einen flächigen Schallwandler auf, der durch Anlegen einer Spannung zumindest bereichsweise zum Schwingen anregbar ist. Dass der Schallwandler flächig ausgestaltet ist, bedeutet dabei, dass zumindest jener Bereich, der durch Anlegen einer Spannung zum Schwingen anregbar ist, der Schwallwandler sich flächig erstreckt. Optional kann sich der flächige Bereich auch in einer Ebene erstrecken. Vorzugsweise sind eine Ober- und eine Unterseite des flächigen Bereichs parallel zueinander.

Erfindungsgemäß ist der Schallwandler so ausgestaltet, dass er in, auf und/oder vor einem runden Fenster oder einem ovalen Fenster oder einem chirurgisch geschaffenen dritten Fenster eines Ohres eines Patienten und/oder in einer Rundfensternische eines Ohres anordenbar ist, so dass er das entsprechende Fenster zumindest teilweise oder vollständig abdeckt. Vorzugsweise erstreckt sich dabei der flächig ausgestaltete Bereich des Schallwandlers über zumindest einen Teil einer Fläche des entsprechenden Fensters. Der Schallwandler kann dabei optional so ausgestaltet sein, dass er im entsprechenden Fenster anordenbar ist, so dass sein schwingungsfähiger Bereich sich in einer Öffnungsebene des entsprechenden Fensters erstreckt. Der Schallwandler deckt also vorzugsweise das entsprechende Fenster zumindest teilweise oder vollständig ab.

Der Schallwandler ist erfindungsgemäß so angeordnet, dass Schwingungen des Schallwandlers Schallschwingungen durch das entsprechende Fenster bewirken. Ist der Schallwandler im entsprechenden Fenster angeordnet, so bewirken Schwingungen des Schallwandlers vorzugsweise Schallschwingungen, die vom entsprechenden Fenster in Richtung der Cochlea ausgehen.

Erfindungsgemäß verläuft der längliche Elektrodenträger im implantierten Zustand durch die Fläche des flächigen Schallwandlers, die sich im implantierten Zustand zumindest zum Teil über das entsprechende Fenster erstreckt. Die entsprechende Fläche wird also in einem Teilbereich von dem Elektrodenträger durchstoßen. In einer optionalen Ausgestaltung der Erfindung kann eine Längsrichtung des Elektrodenträgers in jenem Abschnitt des Elektrodenträgers, in dem dieser den Schallwandler durchläuft, im Wesentlichen senkrecht auf der genannten Fläche des Schallwandlers stehen.

Die Membran des entsprechenden Fensters regeneriert sich nach der Implantation und umschließt den Elektrodenträger dann. Der Aktor verdrängt die Flüssigkeit in der Cochlea über die regenerierte Membran.

In einer vorteilhaften Ausgestaltung des Schallwandlers weist der flächige Schallwandler als Teil seiner genannten Fläche eine Membranstruktur auf. Insbesondere kann vorteilhaft die Membranstruktur die Fläche des Schallwandlers sein. Der flächige Bereich des Schallwandlers ist also in diesem Fall die Membranstruktur. Andere Teile des Schallwandlers, wie beispielsweise eine optionale Aufhängung der Membranstruktur, können eine nichtflächige Ausgestaltung haben.

Vorteilhafterweise weist die Membranstruktur zumindest eine Trägerschicht sowie zumindest eine auf der Trägerschicht angeordnete Piezoschicht auf, die zumindest ein piezoelektrisches Material aufweist. Auf diese Weise kann der Schallwandler durch Anlegen einer Spannung an die Piezoschicht zumindest bereichsweise zum Schwingen anregbar sein. Vorzugsweise ist die Piezoschicht unmittelbar auf der Trägerschicht angeordnet. Bevorzugt erstrecken sich die Trägerschicht und die Piezoschicht parallel zueinander.

In einer vorteilhaften Ausgestaltung kann die beschriebene Membranstruktur in ihrer Fläche durch zumindest eine Schnittlinie, die alle Schichten der Membranstruktur durchtrennt, in ein, zwei oder mehr Segmente unterteilt sein, so dass die Membranstruktur an der Schnittlinie mechanisch entkoppelt ist. Unterteilung der Membranfläche bedeutet dabei, dass die gesamte Membran, also sowohl die zumindest eine Trägerschicht als auch die zumindest eine Piezoschicht sowie gegebenenfalls Elektrodenschichten, durch gemeinsame Schnittlinien unterteilt ist, so dass die Membran an der oder den Schnittlinien mechanisch entkoppelt ist. Mechanische Entkopplung bedeutet dabei, dass zwei durch eine Schnittlinie getrennte Bereiche der Membranstruktur unabhängig voneinander bewegbar sind. Die Unterteilung bzw. Segmentierung der Membranfläche bedeutet also entsprechende Segmentierung der Trägerschicht und entsprechende Segmentierung der Piezoschichten und gegebenenfalls Elektrodenschichten.

Die Segmentierung ermöglicht eine hohe Amplitude einer Schwingung bei sehr kleiner Baugröße, ohne dass durch diese Maßnahme die Kraft zu niedrig wird.

Eine möglichst nahe Ankopplung eines Schallwandlers am runden Fenster (fenestra cochleae) oder ovalen Fenster (fenestra ovalis oder vestibularis) ist für die audiologische Qualität eines mit dem Schallwandler ausgestatteten Hörgerätes, insbesondere als Schallerzeuger, vorteilhaft. Ein vor dem runden oder ovalen Fenster angeordneter Schallwandler ist außerdem von einem implantierenden Chirurgen über den Zugang über äußeren Gehörgang und Trommelfell in relativ kurzer Zeit, eventuell sogar rein ambulant, implantierbar.

Bevorzugt ist daher die Membranstruktur so ausgestaltet, dass der Schallwandler auf, in oder vor einem runden Fenster oder einem ovalen Fenster oder einem chirurgisch geschaffenen dritten Fenster eines Ohres bzw. eines Gehörs so anordenbar ist, dass er dieses Fenster zumindest teilweise oder vollständig abdeckt. Der Schallwandler ist im Falle eines Schallerzeugers mit der Membranstruktur dabei so anordenbar, dass Schwingungen der Membranstruktur Schallschwingungen durch das runde bzw. das ovale Fenster bewirken. Bevorzugt kann die Membranstruktur dabei auch mit der Membran des entsprechenden Fensters in unmittelbarem Kontakt stehen.

Besonders bevorzugt ist der Schallwandler und die Membranstruktur so ausgestaltet, dass der Schallwandler in einer Nische vor dem ovalen oder runden Fenster eines Ohres, d. h. der Rundfensternische, gemessen am Durchschnitt der Bevölkerung oder der Mehrheit der Bevölkerung, einbringbar ist. Dabei kann eine akustische Kopplung zwischen der Membranstruktur und der entsprechenden Fenstermembran einerseits durch Einbringen von Material zwischen die Membranstruktur und die Fenstermembran, beide berührend, hergestellt werden. Bevorzugt ist jedoch, wenn die Membranstruktur so an dem runden oder ovalen Fenster angeordnet wird, dass sie die Membran des entsprechenden Fensters unmittelbar kontaktiert, wobei jedoch erlaubt ist, dass zwischen der eigentlichen Membranstruktur und der entsprechenden Fenstermembran Schichten zur Passivierung bzw. Abdichtung der Membranstruktur angeordnet sind.

Unter Schallschwingungen werden im Sinne der Anmeldung Schwingungen mit Frequenzen verstanden, die vom menschlichen Gehör wahrnehmbar sind, d. h. Schwingungen zwischen ca. 2 Hz und 20.000 bis 30.000 Hz. Die Schallschwingungen sind außerdem geeignet, in einem Medium, insbesondere Luft oder Perilymphe, Schallwellen anzuregen.

Vorteilhaft sind Schallschwingungen durch das entsprechende Fenster erzeugbar. Das kann bedeuten, dass durch den Schallwandler im Innenohr Schallwellen anregbar sind, die vom entsprechenden runden bzw. ovalen Fenster ausgehen. Es können vorteilhaft also vom entsprechenden Fenster ausgehende Schallwellen erzeugt werden, indem die Membranstruktur in, auf oder vor dem entsprechenden Fenster in Schwingung versetzt wird und dadurch die Perilymphe, also ein flüssiges Medium im Innenohr, unmittelbar zur Schwingung anregt oder eine Fenstermembran zur Schwingung anregt, welche dann ihrerseits die Perilymphe anregt.

Vorteilhaft kann die Membranstruktur zumindest eine Trägerschicht und zumindest eine auf der Trägerschicht angeordnete Piezoschicht aufweisen, welche zumindest ein piezoelektrisches Material aufweist. Die Trägerschicht und die Piezoschicht können eine Bimorph-Struktur bilden und so angeordnet und ausgebildet sein, dass die Membranstruktur durch Anlegen einer Spannung, insbesondere einer Wechselspannung, an die Piezoschicht in Schwingung versetzbar ist und/oder dass durch Schwingung der Membran erzeugte Spannungen in der Piezoschicht detektierbar sind. Die Trägerschicht und die Piezoschicht können hierzu mit parallelen Schichtebenen aufeinander oder aneinander angeordnet sein und sollten vorzugsweise direkt oder mittelbar miteinander verbunden sein. Die genannten Schnittlinien durchtrennen vorzugsweise alle Schichten der Membranstruktur.

Vorteilhafterweise wird, um eine gute audiologische Qualität zu gewährleisten, die Membranstruktur so ausgebildet, dass sie eine maximale Auslenkung von 1 bis 5 µm ermöglicht, vorzugsweise von 5 µm. Hierzu ist z. B. bei einer Frequenz v von 4 kHz, einer akustischen Flussimpedanz ZF des runden Fensters von 32 GΩ und einer Fläche A der Membran des runden Fensters von ungefähr 2 mm² eine treibende Kraft von 2 π v Z_{F}A²x = 1,6 10⁻² N nötig. Die durchschnittliche Energie entspricht der Hälfte des Produkts von maximaler Kraft und maximaler Auslenkung, also in diesem Beispiel 4·10⁻⁸ J, um die Leistung zu erhalten. Umgerechnet auf einen Bauraum von z. B. 2 mm³ benötigt man in diesem Beispiel demnach eine Energiedichte von 20 J/m³. Die Segmente können so gestaltet werden, insbesondere bezüglich ihrer Länge, dass die Impedanz optimal ist.

Besonders bevorzugt ist hierzu die Membranstruktur in Dünnschichttechnik ausgeführt. Dünne Schichten sind vorteilhaft, da hohe Felder erforderlich sind, um hohe Energiedichten zu erzeugen, während aber die anlegbaren Spannungen wegen der biologischen Umgebung möglichst niedrig sein sollten. In einer Dünnschichtmembran sind die erforderlichen Energiedichten besonders vorteilhaft erzielbar.

Dabei können vorteilhafterweise insbesondere die Piezoschichten in Dünnschichttechnik hergestellt werden. Hierzu wird für eine herzustellende Piezoschicht der Membranstruktur Piezomaterial in der Dicke der Piezoschicht aufgetragen. Das Auftragen kann über Abscheidetechniken wie Physical-Vapor-Deposition-, Chemical-Vapor-Deposition-Sputtern, Sol-Gel und andere erfolgen. Durch die Herstellung der Piezoschichten durch Abscheiden von Piezomaterial in der gewünschten Dicke lassen sich deutlich dünnere Piezoschichten realisieren als nach dem Stand der Technik, wo fertig gewachsene Piezokristalle auf die Dicke der Piezoschicht abgeschliffen wurden.

Vorzugsweise haben die Piezoschichten eine Dicke von ≤ 20 µm, bevorzugt ≤ 10 µm, besonders bevorzugt ≤ 5 µm und/oder ≥ 0,2 µm, vorzugsweise ≥ 1 µm, bevorzugt ≥ 1,5 µm, besonders bevorzugt = 2 µm. Die Elektrodenschichten haben vorzugsweise eine Dicke von ≤ 0,5 µm, vorzugsweise ≤ 0,2 µm, besonders bevorzugt ≤ 0,1 µm und/oder ≥ 0,02 µm, vorzugsweise ≥ 0,05 µm und besonders bevorzugt ≥ 0,08 µm.

Dünne Schichten des Schallwandlers - sowohl die der Silizium-Balkenstruktur als auch die der Piezoschicht(en) - sorgen dafür, dass bei Auslenkung der Balken nur eine kleine Masse in Bewegung versetzt wird. Die Resonanzfrequenz des Schwingungssystems befindet sich für die beschriebenen Aktorvarianten im oberen Bereich der Frequenzbandbreite des menschlichen Gehörs.

Die Erzeugung der mechanischen Schwingungen des Schallwandlers beruht dabei auf dem Prinzip der elastischen Verformung eines Biegebalkens, wobei die Membran oder Segmente der Membran als Biegebalken betrachtet werden können. Die piezoelektrische Schicht (Piezoschicht) ist dabei durch Anlegen der Spannung und des hierdurch erzeugbaren elektrischen Feldes verkürzbar und/oder verlängerbar. Im Materialverbund aus Trägerschicht und Piezoschicht werden hierbei mechanische Spannungen erzeugt, die zu einer Aufwärtsbiegung des Balkens bzw. der Membranstruktur bei sich verkürzender Piezoschicht führen und zu einer entsprechenden Abwärtsbewegung bei sich verlängernder Piezoschicht. Ob die Piezoschicht sich verlängert oder verkürzt, hängt dabei von der Polarisationsrichtung der Piezoschicht und der Richtung der anliegenden Spannung bzw. des anliegenden elektrischen Feldes ab.

Bei einem einschichtigen Schallwandler kann die beschriebene Trägerschicht eine einzelne Schicht piezoelektrischen Materials tragen. Zusätzlich dazu können Elektroden weitere Komponenten des Schichtaufbaus bilden. Eine Bottom-Elektrode kann dabei direkt oder über einer Barriereschicht auf einem Siliziumsubstrat aufgebracht sein, wohingegen sich eine Top-Elektrode auf der piezoelektrischen Schicht befinden kann. Die Polungsrichtung des piezoelektrischen Materials ist vorzugsweise senkrecht zur Oberfläche der Siliziumstruktur. Wenn nun zwischen Top- und Bottom-Elektrode eine elektrische Spannung angelegt wird und sich eine elektrisches Feld ausbildet, verkürzt oder verlängert sich (je nach Vorzeichen der Spannung) das Piezomaterial in Balkenlängsrichtung durch den transversalen piezoelektrischen Effekt, mechanische Spannungen im Schichtverbund werden erzeugt, und die Balkenstruktur erfährt eine Biegung.

Es ist bevorzugt, wenn die Membranstruktur einen kreisförmigen oder ovalen Umfang hat. Insbesondere ist es hierbei günstig, wenn der Umfang der Membranstruktur dem Umfang des runden oder ovalen Fensters eines Ohres entspricht, so dass die Umfangslinie der Membranstruktur parallel zum Umfang des runden oder ovalen Fensters läuft, wenn der Schallwandler implantiert wird.

Durch eine runde oder leicht ovale Form kann der Schallwandler direkt auf die Membran des runden Fensters aufgesetzt werden. Da die Rundfenstermembran an seiner knöchernen Umrandung als fest eingespannt betrachtet werden kann und dort keine Schwingungsauslenkung zeigt, treten die maximalen Schwingungsauslenkungen im geometrischen Zentrum der Membran auf. Wird der Schallwandler nun mittig auf der Rundfenstermembran platziert, überlagern sich die maximalen Auslenkungen von Schallwandler und Membran, so dass eine gute audiologische Ankopplung und ein großes Schallverstärkungspotential durch den Wandler erreicht wird. Auch ein n-eckiger Umfang der Membranstruktur mit n vorzugsweise ≥ 8 ist möglich.

Insbesondere im Falle eines kreisförmigen Umfangs, aber auch bei anderen Formen der Membranstruktur ist es weiter bevorzugt, wenn die Schnittlinien, welche die Membranfläche in Segmente unterteilen, radial von einem Rand der Membranstruktur in Richtung eines Mittelpunktes der Membran verlaufen. Hierbei müssen die Schnittlinien nicht unmittelbar am Rand starten und nicht bis zum Mittelpunkt reichen, es ist auch hinreichend, wenn die Schnittlinien von der Nähe des Randes bis in die Nähe des Mittelpunktes verlaufen. Sofern allerdings die Schnittlinien den Mittelpunkt nicht erreichen, sollte im Mittelpunkt ein freier Bereich vorliegen, in welchem die Schnittlinien enden, so dass die mechanische Entkopplung der Segmente an jenem dem Mittelpunkt zugewandten Ende gewährleistet ist.

Die Segmente können hierbei so ausgestaltet sein, dass sie kuchenstückförmig sind, also zwei in einem Winkel zueinander verlaufende Ränder als Seitenränder sowie einen Außenrand aufweisen, der am Umfang der Membranstruktur parallel zu diesem Umfang verläuft. Am anderen Ende der Seitenränder, dem Außenrand gegenüber, können die Segmente spitz zulaufen oder so abgeschnitten sein, dass sich um den Mittelpunkt ein freier Bereich ergibt. In einer vorteilhaften Ausgestaltung kann der Elektrodenträger durch diesen freien Bereich verlaufen.

Die Segmente können am äußeren Rand am Rand der Membranstruktur fest angeordnet sein und an den Seitenrändern und gegebenenfalls jenem dem Mittelpunkt zugewandten Rand voneinander unabhängig sein, so dass sie um den äußeren Rand frei schwingen können. Die größte Auslenkung wird hierbei normalerweise an jenem dem Mittelpunkt zugewandten Ende des Segmentes auftreten. Vorzugsweise ist die Zahl der Segmente ≥ 8.

Die Schnittlinien können hierbei radial gerade verlaufen, so dass die Segmente gerade radiale Kanten haben.

Es ist jedoch auch möglich, dass die radial verlaufenden Schnittlinien gekrümmt verlaufen, so dass sich Segmente mit nicht geraden radial verlaufenden Kanten ergeben. Insbesondere können hierdurch Segmente gebildet werden, die in radialer Richtung bogenförmig, wellenförmig oder entlang einer Zickzacklinie verlaufen. Zahlreiche andere Geometrien sind denkbar.

In einer alternativen Ausführungsform der Erfindung kann die Membranstruktur durch zumindest eine Schnittlinie spiralförmig strukturiert sein. Die zumindest eine Schnittlinie verläuft dabei so, dass sich zumindest ein spiralförmiges Segment ergibt, das sich vorzugsweise um einen Mittelpunkt der Membranstruktur windet. Möglich ist es auch, mehrere Schnittlinien vorzusehen, welche die Membranstruktur so unterteilen, dass sich zwei oder mehr spiralförmige Segmente ergeben, die sich vorteilhaft jeweils um den Mittelpunkt der Membranstruktur winden und besonders bevorzugt ineinander verlaufen. Die Spiralstruktur kann eine Öffnung in ihrem Mittelpunkt aufweisen, durch die sich der Elektrodenträger erstrecken kann.

Um die Membranstruktur in Schwingung zu versetzen und/oder um eine Spannung an der Piezoschicht abzugreifen, können zumindest eine erste und zumindest eine zweite Elektrodenschicht an der Membranstruktur angeordnet sein, wobei die zumindest eine Piezoschicht zwischen der ersten und der zweiten Elektrodenschicht angeordnet ist. Die Elektrodenschichten überdecken hierbei vorzugsweise die Piezoschicht und sind mit parallelen Schichtebenen an oder auf der Piezoschicht angeordnet. Vorzugsweise ist die erste oder zweite Elektrodenschicht zwischen der Trägerschicht und der Piezoschicht angeordnet, so dass die Piezoschicht über eine der Elektrodenschichten auf der Trägerschicht angeordnet ist. Besonders bevorzugt bedecken die Piezoschicht und die Elektrodenschichten einander vollständig.

Die Verwendung von Segmentstrukturen erlaubt gegenüber einer unstrukturierten Membran eine höhere Auslenkung, da sich die Balkenelemente dort, wo sie durch die Schnittlinien separiert sind, z. B. im Zentrum der Scheibe, frei verformen können und somit eine konstante Biegung in nur eine Richtung erfahren. Die Verformung einer zusammenhängenden Membran ist hingegen durch eine Richtungsänderung der Krümmung charakterisiert, was zu niedrigeren Auslenkungen führt.

In einer bevorzugten Ausführungsform weist die Membranstruktur eine Mehrzahl von mit parallelen Flächen aufeinander angeordneten Piezoschichten auf, wobei zwischen je zwei benachbarten Piezoschichten eine Elektrodenschicht angeordnet ist. Es ist also auf der Trägerschicht abwechselnd jeweils eine Elektrodenschicht und eine Piezoschicht angeordnet. Elektrodenschichten und Piezoschichten können unmittelbar aufeinander, miteinander verbunden, angeordnet sein oder über ein oder mehrere Zwischenschichten aufeinander angeordnet sein. Mit dieser Ausführungsform lassen sich Schwingungen mit besonders großer Kraft bzw. Leistung erzeugen und Schwingungen besonders genau detektieren.

Bei dieser Wandlermodifikation wechseln sich also im Schichtaufbau Elektroden mit unterschiedlichem elektrischem Potential mit Piezoschichten ab. Auf die Siliziumstruktur folgt zunächst eine Bottom-Elektrode, darauf eine erste Piezoschicht, eine Elektrode mit entgegen gesetztem Potential, eine zweite Piezoschicht, eine Elektrode mit dem Potential der Bottom-Elektrode, usw.

Die Polungsrichtung der einzelnen Piezoschichten kann wie beim Einschichtwandler senkrecht zur Oberfläche der Membranstruktur liegen, allerdings zeigt sie für abwechselnde Piezoschichten in entgegengesetzte Richtung. Das sich zwischen den Elektroden entgegengesetzten Potentials aufbauende elektrische Feld und die für die einzelnen Piezoschichten abwechselnde Polarisierungsrichtung sorgt für eine gemeinsame Längenänderung des gesamten Schichtaufbaus, was wiederum eine Biegung der Siliziumstruktur hervorruft.

Vorteilhafterweise sind die Elektrodenschichten so ausgestaltet bzw. so kontaktiert, dass je zwei benachbarte Elektrodenschichten mit Ladung unterschiedlicher Polarität beaufschlagbar sind. Es ist hierdurch ein elektrisches Feld in den Piezoschichten erzeugbar, das jeweils von einer Elektrodenschicht zur benachbarten Elektrodenschicht verläuft. Auf diese Weise können die Piezoschichten besonders gleichmäßig mit elektrischen Feldern durchsetzt werden. Im Falle einer Schwingungsdetektion können vorzugsweise unterschiedliche Vorzeichen einer an der Piezoschicht entstehenden Spannung jeweils durch benachbarte Elektrodenschichten abgegriffen werden.

In einer weiteren vorteilhaften Ausführungsform der vorliegenden Erfindung können zumindest zwei bandförmige, also längliche Elektroden, die ein Elektrodenpaar bilden, auf der Oberfläche der zumindest einen Piezoschicht oder auf der Oberfläche der Trägerschicht so angeordnet sein, dass sie parallel zur entsprechenden Oberfläche verlaufen und vorzugsweise auch parallel zueinander verlaufen. Die beiden Elektroden eines Elektrodenpaars sind jeweils mit Ladung unterschiedlicher Polarität beaufschlagbar, so dass sich zwischen den Elektroden eines Elektrodenpaars ein elektrisches Feld ausbildet, welches zumindest bereichsweise die Piezoschicht durchsetzt. Sind mehrere Elektrodenpaare vorgesehen, so kann sich auch zwischen Elektroden unterschiedlicher Polarität benachbarter Elektrodenpaare ein elektrisches Feld ausbilden, das die Piezoschicht durchsetzt. Im Fall einer Schwingungsdetektion sind entsprechend durch je eine Elektrode des Elektrodenpaares unterschiedliche Vorzeichen der unten stehenden Spannung kontaktierbar.

Die Leiterbahnstrukturen der bandförmigen Elektroden können vorzugsweise einen rechteckigen Querschnitt haben.

Besonders vorteilhaft ist es, wenn eine Vielzahl von Elektrodenpaaren mit jeweils zwei Elektroden, die mit unterschiedlicher Polarität beaufschlagbar sind, so angeordnet sind, dass die Elektroden der Vielzahl von Elektrodenpaaren parallel zueinander verlaufen. Dabei sollten die Elektrodenpaare außerdem so angeordnet sein, dass jeweils zwei benachbart verlaufende Elektroden mit Ladung unterschiedlicher Polarität beaufschlagbar sind. Auf diese Weise bildet sich zwischen je zwei benachbarten Elektroden ein die Piezoschicht durchsetzendes elektrisches Feld. Für den Fall, dass, wie hier beschrieben, eine Vielzahl von Elektrodenpaaren vorgesehen sind, liegen also eine Vielzahl von Elektroden auf einer Oberfläche der Piezoschicht oder der Trägerschicht vor, die parallel zueinander verlaufen können und mit abwechselnder Polarität nebeneinander angeordnet sein können.

Die Polung des Piezomaterials ist in diesem Fall vorzugsweise nicht homogen über die gesamte Piezoschicht verteilt, vielmehr verläuft die Polarisationsrichtung bevorzugt feldlinienförmig von der negativen zur positiven Elektrode. Wenn im Betrieb des Wandlers die kammförmigen Elektroden mit wechselndem elektrischem Potential beaufschlagt werden, bildet sich entlang der Polarisierungsrichtung des Piezomaterials ein elektrisches Feld aus, entlang dessen sich das Piezomaterial ausdehnt bzw. verkürzt. Dadurch verlängert bzw. verkürzt sich die gesamte Piezoschicht in Balkenlängsrichtung, was zu einer Abwärtsbiegung bzw. Aufwärtsbiegung der Siliziumstruktur führt.

Besonders vorteilhaft ist es, wenn die Elektroden hierbei außerdem parallel zu dem Rand der Membranstruktur verlaufen. Ist also die Membranstruktur kreisförmig, so können die Elektroden bevorzugt konzentrische Kreise um den Mittelpunkt der Membranstruktur bilden. Entsprechend sind bei einer ovalen Membranstruktur auch die Elektroden bevorzugt oval ausgestaltet. Die Elektroden können jeweils entlang des gesamten Umfangs parallel zum Umfang der Membranstruktur verlaufen oder nur auf einem Teil des Umfangs, so dass sie beispielsweise die Form von Kreisumfangsabschnitten haben.

Bandförmige Elektroden lassen sich besonders vorteilhaft über gemeinsame Leiter kontaktieren, wobei eine Mehrzahl der Elektroden durch einen gemeinsamen Leiter kontaktiert wird. So können eine Mehrzahl der Elektroden einer Polarität mit zumindest einem ersten Leiter verbunden sein und Elektroden der anderen Polarität mit zumindest einem zweiten Leiter. Damit die Elektroden unterschiedlicher Polarität abwechselnd angeordnet sind, können die den unterschiedlichen Leitern zugeordneten Elektroden unterschiedlicher Polarität kammförmig ineinandergreifen. Die gemeinsamen Leiter können hierbei die Elektroden der ihnen entsprechenden Polarität schneiden und verlaufen z. B. bei kreisförmigen Elektroden besonders bevorzugt radial.

Auch im Falle einer bandförmigen Ausgestaltung der Elektroden kann die Membranstruktur mehrschichtig ausgestaltet sein. Hierbei ist es zum einen wiederum möglich, dass mehrere Piezoschichten aufeinander angeordnet sind, wobei dann bandförmige Elektroden zwischen jeweils zwei benachbarten Piezoschichten verlaufen können. Die Anordnung der Elektroden entspricht hierbei der oben beschriebenen Anordnung auf der Oberfläche einer Piezoschicht. Möglich ist es aber auch, dass die Membranstruktur zumindest eine Piezoschicht aufweist, die von bandförmigen Elektroden bzw. Elektrodenpaaren in einer oder mehreren Ebenen durchsetzt ist. In diesem Fall verlaufen die Elektroden der Elektrodenpaare im Inneren der entsprechenden Piezoschicht. Die verschiedenen Möglichkeiten der Anordnung entsprechen auch hier jenen der oben genannten Anordnung auf der Oberfläche der Piezoschicht.

Diese Variante des Schallwandlers weist gegenüber der vorigen Lösung eine dickere Piezoschicht auf, die von mehreren Lagen kammförmiger Elektroden durchzogen werden kann. Die Polarisierung im Piezomaterial verläuft wiederum feldlinienförmig von den negativen zu den positiven Leiterbahnelektroden. Bei anliegender Spannung bildet sich entlang der Polarisierungsrichtung ein elektrisches Feld aus, das zu einer Ausdehnung bzw. Verkürzung des Piezomaterials entlang der Feldlinien und zu einer Abwärtsbiegung bzw. Aufwärtsbiegung der Balkenstruktur führt.

Im Falle spiralförmiger Segmente können bandförmige Elektroden entlang der Längsrichtung der Segmente angeordnet sein. Vorzugsweise reicht hier ein Elektrodenpaar aus.

Da der Schallwandler in einer biologischen Umgebung zum Einsatz kommt, ist es vorteilhaft, wenn die Spannung, mit welcher die Elektroden beaufschlagt werden, kleiner ist als 3 Volt, bevorzugt kleiner als 2 Volt, besonders bevorzugt kleiner als 1,3 Volt. Alternativ oder zusätzlich ist es auch möglich, die Elektroden flüssigkeitsdicht und/oder elektrisch isolierend zu kapseln, so dass sie mit einer den Schallwandler gegebenenfalls umgebenden Flüssigkeit nicht in Kontakt kommen. Eine so dichte Kapselung wird jedoch eine derart hohe akustische Impedanz aufweisen, dass mit beträchtlichen audiologischen Verlusten zu rechnen ist.

Da der piezoelektrische Effekt im betrachteten Bereich proportional zur Stärke des elektrischen Feldes ist, welches das Material durchdringt, können durch Verwendung sehr dünner piezoelektrischer Schichten bei sehr kleinem Abstand der Elektroden so hohe Felder erzeugt werden (das elektrische Feld berechnet sich im homogenen Fall als Quotient aus anliegender Spannung und Abstand der Elektroden), dass der Piezoeffekt ausreicht, um die für die Anregung des runden Fensters notwendigen Schwingungsauslenkungen und Kräfte zu erreichen.

Die Trägerschicht kann Silizium aufweisen oder daraus bestehen. Als Piezomaterialien kommen u.a.
PbZrₓTi₁₋ₓO3 mit vorzugsweise 0,45 < x < 0,59, besonders bevorzugt mit Dotierungen von beispielsweise La, Mg, Nb, Ta, Sr und dergleichen, vorzugsweise mit Konzentrationen zwischen 0,1 und 10%, in Frage. Auch weitere feste Lösungen mit PbTiO₃, wie beispielsweise Pb(Mg_{1/3}, Nb_{2/3})O₃, Pb(Sn_{1/3}Nb_{2/3})O₃, kommen in Frage. Mögliche Materialien sind auch bleifreie Materialien, die KNbO₃, NaNbO₃ enthalten, Dotierungen mit Li, Ta, etc., Bihaltige Piezoschichten, Aurivilliusphasen mit Ti, Ta, Nb, ferner auch Perovskitphasen, wie BiFe₃. Auch klassische Dünnschichtmaterialien, wie AIN und ZnO sind möglich.

Silizium als Trägermaterial für die Piezoschichten ermöglicht das Herstellen der scheibenförmigen Struktur und der kuchenstückförmigen Biegebalken mit den Strukturierungstechniken der Mikrosystemtechnik. Es können bekannte und erprobte Beschichtungs- und Ätzverfahren zur Herstellung von Balken, Elektroden und Piezoschicht verwendet werden, z. B. Sol-Gel-Techniken, Sputter-Verfahren, chemisches Ätzen, lonenätzen, etc. Weiterhin erlauben die Verfahren der Mikrosystemtechnik ein Parallelisieren des Fertigungsprozesses; aus einem Silizium-Wafer lässt sich in einem Fertigungsdurchgang eine Vielzahl von Schallwandlern herstellen. Dies ermöglicht eine kostengünstige Produktion.

Die zumindest eine Piezoschicht hat vorzugsweise eine Dicke von ≤ 20 µm, vorzugsweise ≤ 10 µm, besonders bevorzugt ≤ 5 µm und/oder ≥ 0,2 µm, vorzugsweise ≥ 1 µm, bevorzugt ≥ 1,5 µm, besonders bevorzugt = 2 µm. Die Elektrodenschichten haben jeweils vorzugsweise eine Dicke von ≤ 0,5 µm, vorzugsweise ≤ 0,2 µm, besonders bevorzugt ≤ 0,1 µm und/oder ≥ 0,02 µm, vorzugsweise ≥ 0,05 µm, besonders bevorzugt ≥ 0,08 µm. Ein Durchmesser der Membranstruktur ist vorzugsweise ≤ 4 mm, bevorzugt ≤ 3 mm, besonders bevorzugt ≤ 2 mm und/oder ≥ 0,2 mm, vorzugsweise ≥ 0,5 mm, bevorzugt ≥ 1 mm, besonders bevorzugt = 1,5 mm, und besonders bevorzugt so gewählt, dass der Schallwandler in geeigneter Weise vor dem runden oder ovalen Fenster eines Ohres anordenbar ist. Bevorzugt ist der Schallwandler in der Rundfensternische eines Ohres anordenbar, wobei deren Abmessungen als jene der Mehrheit oder des Durchschnitts der Bevölkerung im Geltungsbereich der vorliegenden Schrift verstanden werden können.

Der erfindungsgemäße Schallwandler kann durch unmittelbares Auflegen der Membranfläche auf einer Membran des runden oder ovalen Fensters direkt angekoppelt werden. Da sich die maximale Schwingungsauslenkung des Wandlers in der geometrischen Mitte der Scheibe mit der maximalen Schwingung der Membran im Zentrum des runden Fensters überlagert, ist eine gute audiologische Ankopplung mit hohem Schallverstärkungspotential möglich.

Erfindungsgemäß kann der Schallwandler auch mehrere wie oben beschriebene Membranstrukturen aufweisen. Diese Membranstrukturen sind dabei gleich strukturiert und parallel zueinander so übereinander angeordnet, dass gleiche Segmente der Struktur bzw. die Schnittlinien der Membranstrukturen übereinander liegen. Gleiche Segmente sind dann so miteinander gekoppelt, dass sich eine Auslenkung und/oder Kraftausübung eines der Segmente auf die benachbarten Segmente überträgt. Die Membranstrukturen können dabei so übereinander angeordnet sein, dass bei Anlegen einer Spannung einer gegebenen Polung an den Schallwandler alle Segmente in die gleiche Richtung ausgelenkt werden. Die Membranstrukturen sind hierbei gleich orientiert. In diesem Fall kann eine Gesamtkraft realisiert werden, die höher ist als die einer einzelnen Membranstruktur. Es ist auch möglich, die Membranstrukturen so aufeinander anzuordnen, dass benachbarte Membranstrukturen jeweils umgekehrt orientiert sind, so dass sich bei Anlegen einer Spannung einer gegebenen Polung benachbarte Membranstrukturen jeweils in unterschiedliche Richtung auslenken. In diesem Fall lässt sich eine Gesamtauslenkung realisieren, die größer ist als die einer einzelnen Membranstruktur.

Die Ausführungsformen der Erfindung können speziell an die Anforderungen eines implantierbaren Hörgerätes mit einer audiologischen Anregung des runden oder ovalen Fensters im Mittelohr angepasst sein. Bevorzugt ist der Schallwandler ein Schallerzeuger. Es ist auch möglich, klassische Hörgeräte, Hörgeräte, die direkt auf dem Trommelfell aufsitzen, oder sonstige Miniaturlautsprecher, wie beispielsweise in Kopfhörern, mit dem erfindungsgemäßen Schallwandler auszustatten. Der Schallwandler ist außerdem als Sensor einsetzbar und ermöglicht, aus einem Schallsignal ein elektrisches Signal zu generieren. Der Schallwandler kann also auch als Mikrofon zum Einsatz kommen.

In einer vorteilhaften Ausgestaltung der Erfindung kann der Schallwandler im Mittelohr einer Person so fixierbar sein, dass Schwingungen des Schallwandlers auf eine Flüssigkeit in der Hörschnecke übertragbar sind. Bevorzugterweise befindet sich zwischen dem Schallwandler und der Flüssigkeit kein Luftvolumen, so dass eine gute Ankopplung des Schallwandlers an die Flüssigkeit in der Hörschnecke möglich wird.

In einer vorteilhaften Ausgestaltung der Erfindung kann der längliche Elektrodenträger durch eine Mitte der Fläche des flächigen Schallwandlers verlaufen. Ist die schwingende Fläche des Schallwandlers wie vorstehend beschrieben segmentiert, so kann hierzu eine Segmentierung vorgesehen sein, die in der Mitte des Schallwandlers eine Öffnung aufweist, in der keine Membranstruktur vorliegt.

Vorstehend wurden betreffend den Schallwandler einige dieser Ausgestaltungen beschrieben. Hat der Schallwandler und/oder seine Membran einen kreisförmigen Umfang, so kann diese Öffnung im Mittelpunkt des Kreises vorliegen. Besonders vorteilhaft ist in diesem Falle eine Segmentierung mit radialen Schnittlinien, wie beispielsweise die oben beschriebene kuchenstückförmige Segmentierung.

Alternativ kann der längliche Elektrodenträger exzentrisch durch die Fläche des flächigen Schallwandlers verlaufen. In diesem Fall durchtritt also der Elektrodenträger die Fläche des Schallwandlers abseits des Mittelpunktes dieser Fläche, jedoch umgibt vorteilhafterweise die Fläche des Schallwandlers den Elektrodenträger vollständig. Für eine solche Durchführung des Elektrodenträgers durch die Fläche des Schallwandlers weist die Fläche vorzugsweise eine entsprechende exzentrische Öffnung auf. Auch für diese Ausführungsform kann vorteilhafterweise die Fläche des Schallwandlers mit radialen Schnittlinien separiert sein. In diesem Falle entstehen jedoch Membranabschnitte, die unterschiedlich lang sind. Jene Membranstücke, in deren Richtung der Elektrodenträger gegenüber der Mitte der Fläche verschoben ist, sind in radialer Richtung kürzer, während gegenüberliegende Segmente in radialer Richtung länger sind. Insbesondere können die Segmente kürzer und länger als die entsprechenden Segmente bei der oben beschriebenen zentrischen Durchführung des Elektrodenträgers durch die Membran sein.

In einer besonders vorteilhaften Ausgestaltung der Erfindung kann der längliche Elektrodenträger die Fläche des flächigen Schallwandlers in einer Einkerbung am Rand der Fläche des flächigen Schallwandlers durchtreten. Eine Einkerbung der Fläche des Schallwandlers ist hierbei ein nicht von der Fläche abgedeckter Bereich, der sich vom Rand der Fläche aus konkav in die Fläche des Schallwandlers hinein erstreckt. In einer vorteilhaften Ausgestaltung der Erfindung kann der Elektrodenträger auf zumindest einem Teil seines Umfangs an einem Rand der Einkerbung anliegen. Bevorzugterweise ist eine radiale Tiefe der Einkerbung gleich einem Durchmesser des länglichen Elektrodenträgers. Auf diese Weise kann der Elektrodenträger mit einem minimalen Verlust schwingungsfähiger Fläche durch den Schallwandler hindurchgeführt werden.

Der längliche Elektrodenträger ist vorzugsweise eine längliche Struktur, an welcher eine Mehrzahl oder Vielzahl von Elektroden in einer Längsrichtung des Elektrodenträgers nebeneinander angeordnet sind. Die Elektroden sind dabei vorzugsweise so angeordnet, dass sie im implantierten Zustand für bestimmte Frequenzen zuständige Sinneszellen im Ohr der das Implantat tragenden Person anregen können. Die genaue Position wird dabei vorzugsweise individuell an die anatomischen Gegebenheiten der entsprechenden Person angepasst.

Bevorzugterweise ist der längliche Elektrodenträger eine Cochlea-Implantatelektrode, deren Länge so ausgestaltet ist, dass mit ihr nur Frequenzen höher als eine bestimmte Schwellfrequenz anregbar sind. Hierzu kann der Elektrodenträger verkürzt sein, so dass er sich nicht bis zur gesamten Tiefe der Schnecke in diese erstreckt, sondern nur bis zu einer bestimmten Position, die der Grenzfrequenz entspricht. In der Schnecke sind die Sinneszellen so angeordnet, dass tiefer in der Schnecke vorliegende Sinneszellen tiefere Frequenzen erfassen als weiter außen liegende Sinneszellen. Durch eine Begrenzung der Länge des Elektrodenträgers kann daher die durch die Elektroden angeregte Frequenz nach unten begrenzt werden. Bevorzugterweise ist die Länge des Elektrodenträgers so ausgestaltet, dass mit den Cochlea-Implantatelektroden dieses Elektrodenträgers im implantierten Zustand nur Frequenzen ≥ 1000 Hz, vorzugsweise ≥ 1300 Hz, besonders bevorzugt ≥ 1500 Hz, anregbar sind.

In einer vorteilhaften Ausgestaltung der Erfindung kann der Schallwandler gegenüber dem Elektrodenträger in einer Längsrichtung des Elektrodenträgers verschiebbar sein. Hierdurch kann bei der Implantation des elektroakustischen Implantats zunächst der Elektrodenträger bis zur vorgesehenen Tiefe in die Cochlea eingeführt werden. Es kann dann vorteilhaft im Anschluss der Aktor auf der Elektrode bis zum entsprechenden Fenster vorgeschoben werden, und die Aktorstirnfläche in direkten Kontakt zur Cochlea gebracht werden. Sind beide Komponenten, also Schallwandler und Elektrodenträger, am vorgesehenen Ort untergebracht, können sie mit einem Befestigungselement, wie beispielsweise einem Clip oder einer Klammer, oder alternativ mit Bindegewebe, fixiert werden.

In einer vorteilhaften Ausgestaltung der Erfindung kann der Schallwandler gegenüber dem Elektrodenträger verkippbar sein. Es kann hierbei also vorteilhaft die Aktorstirnfläche um ein oder zwei Achsen senkrecht zur Längsachse des Elektrodenträgers gekippt werden. Hierdurch kann sichergestellt werden, dass der Aktor plan auf der Fläche der entsprechenden Fenstermembran aufliegt, ohne dass der Elektrodenträger im rechten oder in einem fixen Winkel in die Cochlea eindringen muss. Vorteilhaft kann eine solche Verkippbarkeit erzielt werden, indem zwischen Aktor und Elektrodenträger ein Kugelgelenk vorgesehen wird. Das Kugelgelenk kann beispielsweise als kugelförmige Verdickung des Elektrodenträgers ausgestaltet sein. Alternativ kann eine Verkippung auch dadurch erreicht werden, dass der Elektrodenträger mit einem Spiel in radialer Richtung bezüglich der Längsachse des Elektrodenträgers realisiert wird, und der entstehende Zwischenraum zwischen dem Schallwandler und dem Elektrodenträgers mit einem flexiblen Material, wie beispielsweise Silikon, gefüllt wird. In diesem Fall ist also vorteilhafterweise die Öffnung in der Fläche des Schallwandlers größer als der Durchmesser des Elektrodenträgers. Der Elektrodenträger ist also zumindest bereichsweise von einer Wand der Öffnung beabstandet. In diesem beabstandeten Bereich kann das genannte flexible Material vorgesehen sein.

Das erfindungsgemäße elektroakustische Implantat hat verbesserte akustische Eigenschaften gegenüber einer akustischen Anregung mit einem herkömmlichen Hörgerät wie aus dem Stand der Technik bekannt. Der Schallwandler kann vorteilhafterweise so ausgestaltet sein, dass er eine hohe Resonanzfrequenz aufweist und dadurch für die relevanten niedrigen Frequenzen ein flaches Übertragungsverhalten bei hohen Schwingungsauslenkungen garantiert. Hierdurch und durch eine vorteilhafte Implantation des Implantats in direkten Kontakt zur Cochlea kann ein verzerrungsfreier Höreindruck bis hin zu hohen Verstärkungen erreicht werden, die einem äußeren Höreindruck von 120 dB SPL entsprechen können. Durch eine vorteilhafte örtliche Trennung von Mikrofon (beispielsweise außen am Kopf, über der Ohrmuschel) und dem Schallwandler (vorteilhaft implantiert ins Mittelohr) kann die Gefahr von Rückkopplungen deutlich reduziert werden. Insgesamt ergibt sich gegenüber dem Stand der Technik eine überlegene Tonqualität für die relevanten tiefen Frequenzen und dadurch ein verbessertes Sprachverstehen für die Patienten.

Das beschriebene Hybridsystem zeichnet sich außerdem durch einen kaum erhöhten Operationsaufwand gegenüber der Implantation eines reinen Cochleaimplantats aus, insbesondere wenn Elektrode und Aktor an derselben Stelle der Cochlea platziert werden.

Ein weiterer Vorteil des Konzeptes ist die Miniaturisierung und Reduzierung der äußerlich nicht sichtbaren Komponenten. Durch Wegfall des konventionellen Hörgeräts kann der Gehörgang frei bleiben. Die restlichen äußeren Komponenten, wie Mikrofon, Akku und Soundprozessor, können miniaturisiert in einem einzigen Gehäuse untergebracht werden und nahezu unsichtbar im Haar getragen werden.

Im Folgenden soll die Erfindung beispielhaft anhand einiger Figuren erläutert werden. Gleiche Bezugszeichen entsprechen dabei gleichen oder entsprechenden Merkmalen. Die in den Beispielen beschriebenen und gezeigten Merkmale können auch unabhängig vom entsprechenden Beispiel realisiert sein und zwischen unterschiedlichen Beispielen miteinander kombiniert werden.

Es zeigt
- Fig. 1: ein erfindungsgemäßes elektroakustisches Implantat mit zentrisch durch einen Schallwandler geführtem Elektrodenträger,
- Fig. 2: einen Schnitt durch das in Figur 1 gezeigte Implantat,
- Fig. 3: ein erfindungsgemäßes elektroakustisches Implantat mit einem exzentrisch durch den Schallwandler geführten Elektrodenträger,
- Fig. 4: einen Schnitt durch das in Figur 3 gezeigte Implantat,
- Fig. 5: ein erfindungsgemäßes elektroakustisches Implantat mit einem durch eine Einkerbung im Schallwandler verlaufenden Elektrodenträger,
- Fig. 6: das Prinzip der Auslenkung einer Membranstruktur, wie sie im erfindungsgemäßen Schallwandler zum Einsatz kommen kann,
- Fig. 7: einen Schnitt durch Membranstrukturen, wie sie im erfindungsgemäßen Schallwandler zum Einsatz kommen können,
- Fig. 8: einen Schnitt durch einen Schallwandler mit einer zwischen zwei Elektrodenschichten angeordneten Piezoschicht,
- Fig. 9: eine Aufsicht auf einen Schallwandler mit bandförmigen Elektroden,
- Fig. 10: einen Schnitt durch einen Schallwandler mit auf der Piezoschicht angeordneten bandförmigen Elektroden, und
- Fig. 11: möglichen Anordnungen des erfindungsgemäßen elektroakustischen Implantats im Ohr.

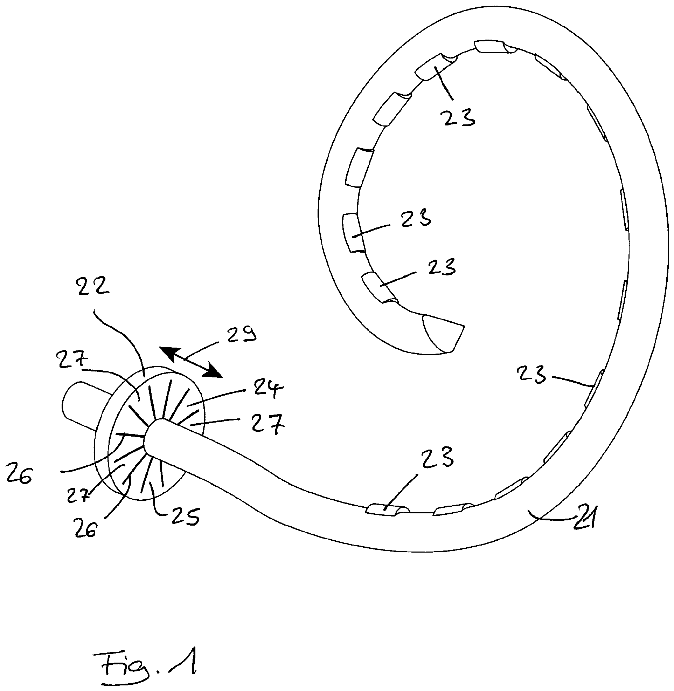

Figur 1 zeigt ein erfindungsgemäßes elektroakustisches Implantat, das einen länglichen Elektrodenträger 21 und einen flächigen Schallwandler 22 aufweist. Der längliche Elektrodenträger 21 weist eine Vielzahl von Stimulationselektroden 23 auf. Der Elektrodenträger 21 ist in eine Hörschnecke einer Person einführbar. Der flächige Schallwandler 22 ist durch Anlegen einer Spannung zumindest bereichsweise zum Schwingen anregbar. Der Schallwandler 22 ist so ausgestaltet, dass er in, auf und/oder vor einem runden Fenster oder einem ovalen Fenster oder einem chirurgisch geschaffenen dritten Fenster eines Ohres und/oder in einer Rundfensternische eines Ohres so anordenbar ist, dass er das entsprechende Fenster zumindest teilweise oder vollständig abdeckt. Schwingungen des Schallwandlers 22 können dann Schallschwingen durch das entsprechende Fenster bewirken, in, auf und/oder vor welchem der Schallwandler 22 angeordnet ist.

Der Elektrodenträger 21 verläuft durch eine Fläche des flächigen Schallwandlers 22. Die Fläche 24 ist jene Fläche, mit der der Schallwandler, wenn er vor dem entsprechenden Fenster angeordnet ist, zumindest zum Teil das entsprechende Fenster abdeckt.

Im gezeigten Beispiel weist der Schallwandler 22 als schwingende Fläche 24 eine segmentierte Membran 25 auf, die durch alle Schichten der Membranstruktur 25 durch trennende Schnittlinien 26 in eine Vielzahl von Segmenten 9a, 9b, 9c unterteilt ist, so dass die Membranstruktur an der Schnittlinie mechanisch entkoppelt ist.

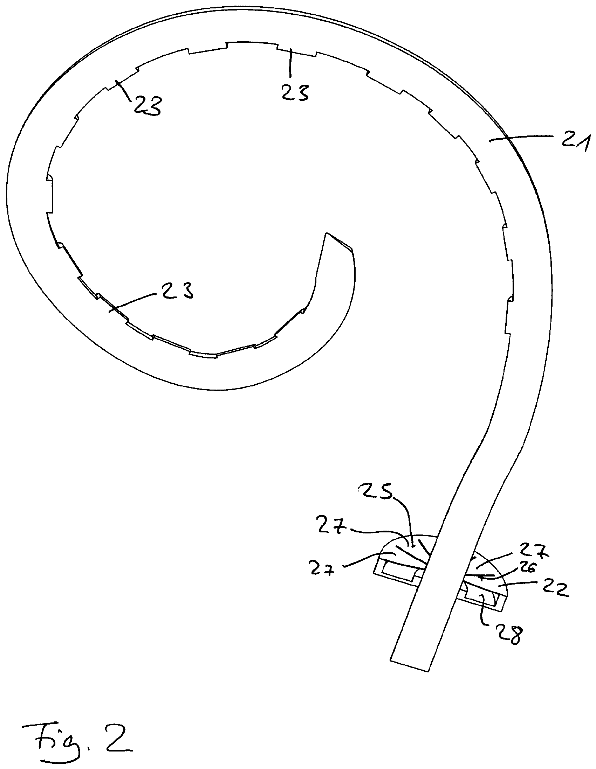

Figur 2 zeigt einen Schnitt durch das in Figur 1 gezeigte elektroakustische Implantat. Die zu Figur 1 gemachten Äußerungen gelten daher für Figur 2 entsprechend. Im Schnitt durch den Schallwandler 22 ist zu erkennen, dass im gezeigten Beispiel die Membranstruktur 25 durch eine Trägerstruktur 28 getragen wird. Die Membranstruktur 25 und die Trägerstruktur 28 sind im gezeigten Beispiel kreisförmig und haben den gleichen Radius. Die Trägerstruktur 28 weist eine ringförmige Fläche auf, die von der Membranstruktur 25 beabstandet ist. Die ringförmige Fläche wird von einem Rand 6 umgeben, der die Membranstruktur 25 an deren Rand trägt. Die Trägerstruktur 28 weist außerdem einen inneren Ring 27 auf, der die ringförmige Fläche in Richtung ihres Mittelpunktes begrenzt und eine Öffnung umschließt, durch welche der Elektrodenträger 21 durch den Schallwandler 22 hindurchtritt. Dieser innere Ring 27 kann den Elektrodenträger 21 berühren und dafür sorgen, dass der Schallwandler 22 fest mit dem Elektrodenträger 21 verbunden ist oder auf dem Elektrodenträger gleiten kann, wie dies in Figur 1 mit dem Doppelpfeil 29 angedeutet ist.

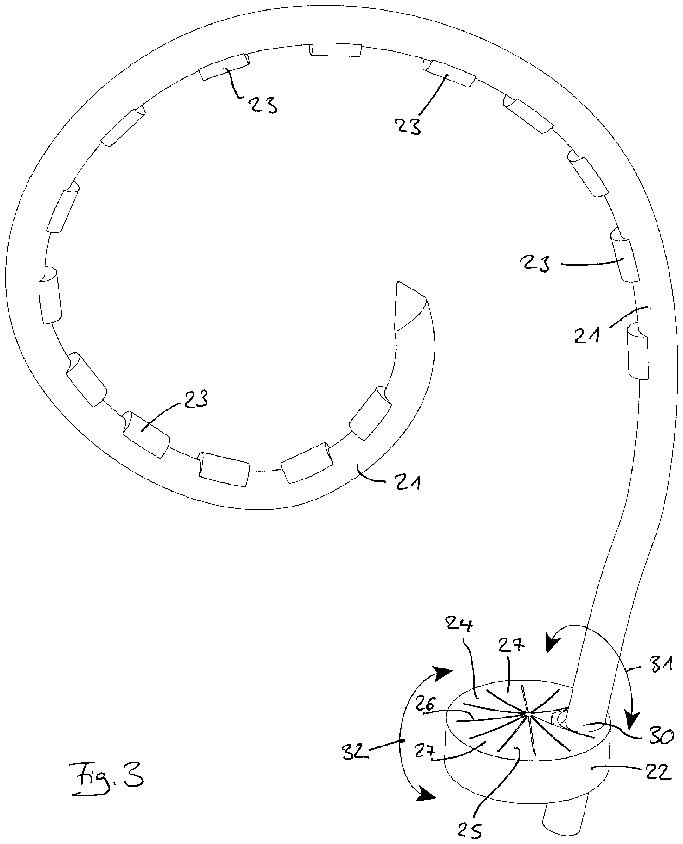

Figur 3 zeigt ein weiteres Ausführungsbeispiel eines elektroakustischen Implantats gemäß der vorliegenden Erfindung. Wiederum wird ein Elektrodenträger 21 durch einen Schallwandler 22 hindurchgeführt, wobei der Elektrodenträger 21 eine Vielzahl von Elektroden 23 trägt. Der Schallwandler weist wiederum eine Membran 25 auf, die durch Schnittlinien 26 in eine Vielzahl von Segmenten 9a, 9b unterteilt ist. Auch hier verlaufen die Schnittlinien 26 radial und durchtrennen alle Schichten der Membran 25, so dass die Segmente 9a, 9b entlang der Schnittlinie 26 mechanisch entkoppelt sind. Die zu Figur 1 und 2 gemachten Aussagen gelten hier entsprechend.

Anders als in den Figuren 1 und 2 ist jedoch in Figur 3 der Elektrodenträger 21 exzentrisch durch den Schallwandler 22 hindurchgeführt, d. h. mit einem Abstand größer als Null zum Mittelpunkt der Membran 25 des Schallwandlers 22.

Im gezeigten Beispiel verläuft der Elektrodenträger 21 durch eine Öffnung 30 in der Membranstruktur 25. Die Öffnung 30 wie auch der Elektrodenträger 21 haben einen kreisförmigen Querschnitt, jedoch ist der Durchmesser des Elektrodenträgers 21 etwas geringer als der Durchmesser der Öffnung 30. Auf diese Weise ist der Schallwandler 22 gegenüber dem Elektrodenträger 21 um zwei zueinander senkrechte Achsen verkippbar, wie dies durch die Pfeile 31 und 32 angedeutet wird.

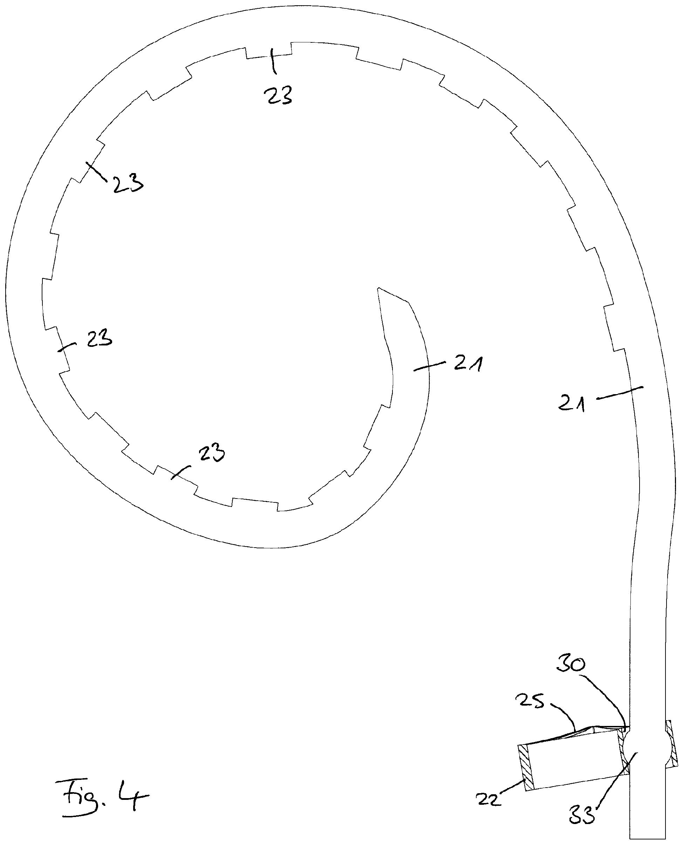

Figur 4 zeigt einen Querschnitt durch ein elektroakustisches Implantat, wie es in Figur 3 gezeigt ist. Im in Figur 4 gezeigten Beispiel weist wiederum die Öffnung 30 einen etwas größeren Durchmesser auf als der Elektrodenträger 21 über den größten Teil seiner Länge. Der Elektrodenträger 21 weist jedoch innerhalb der Öffnung 30 eine kugelförmige Verdickung 33 auf. Dabei ist der Durchmesser der kugelförmigen Verdickung 33, also der Kugeldurchmesser, gleich dem inneren Durchmesser der Öffnung 30. Die Öffnung 30 ist dabei zylinderförmig ausgestaltet, und die kugelförmige Verdickung 33 berührt die innere Zylinderwand der Öffnung 30. Hindurch kann der Elektrodenträger 21 gegenüber dem Schallwandler 25 gekippt werden.

Die in Figur 3 gezeigte Verkippbarkeit des Schallwandlers 22 gegenüber dem Elektrodenträger 21 kann mit einer zylinderförmigen Öffnung 30 auch alternativ dadurch erreicht werden, dass der Elektrodenträger 21 einen konstanten Durchmesser innerhalb der Öffnung 30 hat und im Bereich zwischen der Innenwand der Öffnung 30 und der Oberfläche des Elektrodenträgers 21 ein elastisches Material angeordnet ist. Auch hierdurch wird eine Kippung des Elektrodenträgers 21 gegenüber dem Schallwandler 22 ermöglicht.

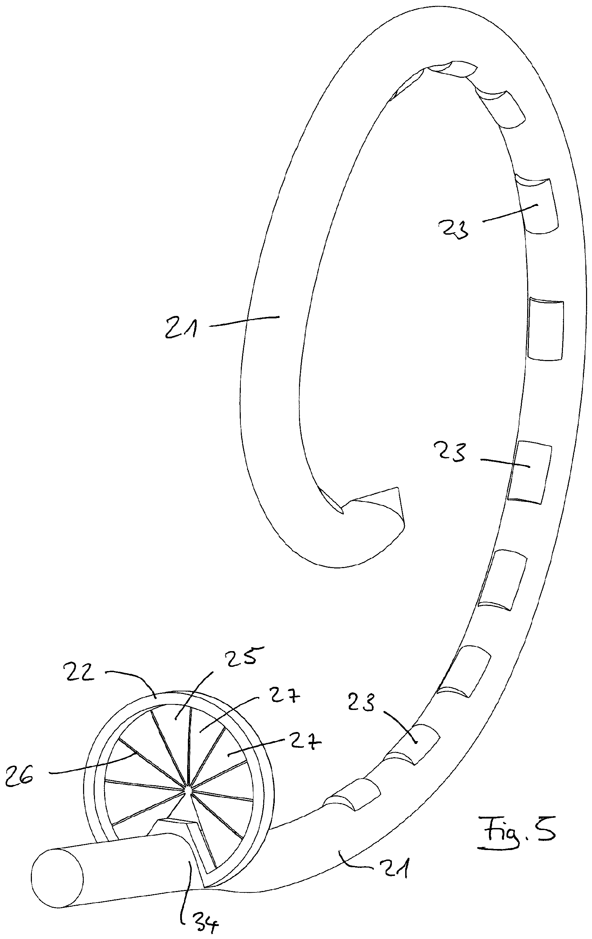

Figur 5 zeigt eine weitere beispielhafte Ausgestaltung eines erfindungsgemäßen elektroakustischen Implantats. Der Aufbau dieses Implantats entspricht dem in den Figuren 1 bis 4 gezeigten, jedoch mit dem Unterschied, dass in den Figuren 1 bis 4 der Elektrodenträger 21 durch eine Öffnung im Schallwandler 22 durch diesen hindurchgeführt wird, die von der Fläche 24 des Schallwandlers 22 vollständig umgeben wird. Im in Figur 5 gezeigten Beispiel wird hingegen der Elektrodenträger 21 durch eine Einkerbung 34 im Schallwandler 22 durch diesen hindurchgeführt. Der äußere Rand des Schallwandlers 22 folgt der konkaven Einkerbung 34, so dass der Elektrodenträger 21 außerhalb des Randes, jedoch weitgehend innerhalb einer durch den Schallwandler 22 beschriebenen Kreisfläche durch den Schallwandler 22 hindurchtritt. Wie in den anderen Beispielen ist auch in Figur 5 die Membran 25 mittels radialer Schnittlinien 26 in eine Vielzahl von Segmenten 9a, 9b unterteilt. Im Bereich der Einkerbung 34 ist jedoch im gezeigten Beispiel kein Segment 9a, 9b der Membranstruktur 25 vorgesehen.

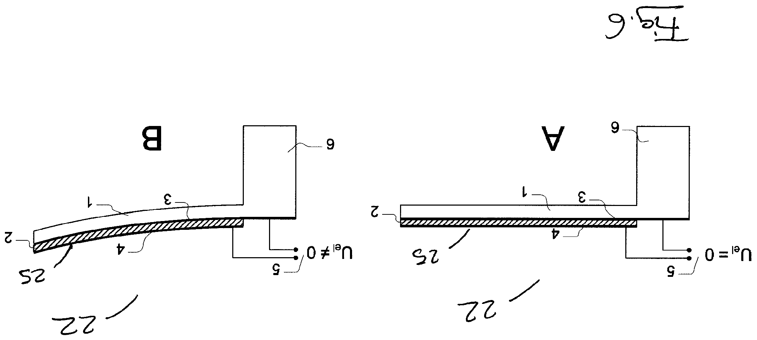

Figur 6 zeigt den prinzipiellen Aufbau eines Schallwandlers 22 zur Erzeugung von Schallschwingungen, der in ein Ohr einsetzbar ist. Im gezeigten Beispiel ist auf einer Trägerschicht 1, beispielsweise einer Siliziumschicht 1, eine Membranstruktur 25 angeordnet, die eine Piezoschicht 2 sowie zwei Elektrodenschichten 3 und 4 aufweist. Die Trägerschicht 1 (elastische Schicht 1) kann dabei z. B. etwa ein- bis zweimal so dick sein wie die piezoelektrische Schicht. Zwischen den Elektrodenschichten 3 und 4 ist mittels einer Spannungsquelle 5 eine Spannung anlegbar oder mittels eines geeigneten Detektors eine Spannung detektierbar. Im gezeigten Beispiel ist auf der Trägerschicht 1 zunächst die eine der Elektrodenschichten 3 angeordnet, auf welcher dann die Piezoschicht 2 angeordnet ist. Auf jener der die Elektrodenschicht 3 kontaktierenden Seite gegenüberliegenden Seite der Piezoschicht 2 ist die zweite Elektrodenschicht 4 angeordnet. Durch Anlegen einer Spannung mittels der Spannungsquelle 5 sind die Elektrodenschichten 3 und 4 mit entgegengesetzter Polarität aufladbar, so dass zwischen den Elektrodenschichten 3 und 4 ein elektrisches Feld entsteht, welches die Piezoschicht 2 durchsetzt.

Figur 6A zeigt den Zustand des Schallwandlers 22 für den Fall, dass keine Spannung angelegt ist. Die Trägerschicht 1, die Piezoschicht 2 und die Elektrodenschichten 3 und 4 erstrecken sich hierbei in einer Ebene, sind also flach. Wird nun, wie in Figur 6B gezeigt, eine Spannung mittels der Spannungsquelle 5 zwischen den Elektrodenschichten 3 und 4 angelegt, so durchsetzt ein elektrisches Feld die Piezoschicht 2. Die Piezoschicht 2 verkürzt sich hierdurch, wodurch sich die gesamte Membranstruktur 25 der Trägerschicht 1, der Elektrodenschichten 3 und 4 sowie der Piezoschicht in Richtung der Piezoschicht nach oben biegt. Wird die Spannung 5 umgepolt, dehnt sich die Piezoschicht 2 aus und die Membranstruktur biegt sich von der Piezoschicht 2 weg. Wird an der Spannungsquelle 5 eine Wechselspannung angelegt, so kann die Membranstruktur in Schwingung versetzt werden.

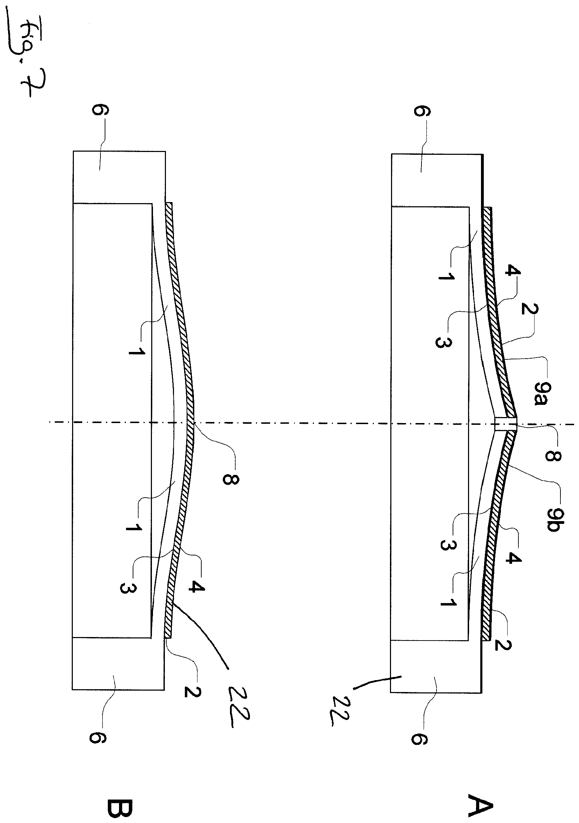

Figur 7 zeigt zwei mögliche Ausführungsformen des erfindungsgemäßen Schallwandlers 22 im Vergleich. Die in Figur 7A gezeigte Ausführungsform entspricht der in Figur 6 gezeigten, wo die Membranstruktur in Segmente 9a, 9b unterteilt ist. In der in Figur 7B gezeigten Ausführungsform liegt hingegen eine unsegmentierte Membranstruktur 25 vor. Die in Figur 7A gezeigte segmentierte Ausführungsform erlaubt hierbei gegenüber der unstrukturierten in Figur 7B gezeigten Membran eine höhere Auslenkung, da sich die beiden Elemente 9a, 9b im Zentrum 8 der kreisförmigen Membran frei verformen können und daher in Richtung vom Rand 6 zur Mitte 8 eine konstante Krümmung in nur eine Richtung erfahren. Bei der in Figur 7B gezeigten unsegmentierten Membran ist die Auslenkung in der Mitte 8 geringer. Darüber hinaus ändert sich die Krümmung der Membran vom Rand 6 in Richtung der Mitte 8 und ändert ihr Vorzeichen. Andererseits erleichtert hingegen die Figur 7B einen gas- und flüssigkeitsdichten Abschluss einer Öffnung durch den erfindungsgemäßen Schallwandler.

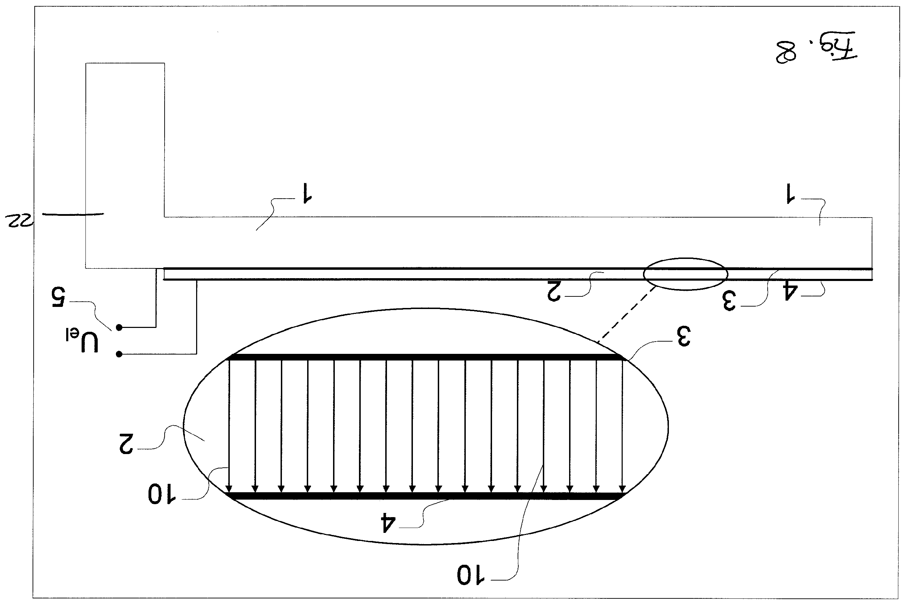

Figur 8 zeigt einen Schnitt durch einen erfindungsgemäßen Schallwandler 22, bei welchem eine piezoelektrische Schicht 2 zwischen einer Elektrodenschicht 3 und einer Elektrodenschicht 4 angeordnet ist. Die Ausführungsform entspricht im Wesentlichen der in Figur 6 gezeigten. Mittels einer Spannungsquelle 5 ist eine Spannung zwischen den Elektrodenschichten 3 und 4 anlegbar, welche ein die piezoelektrische Schicht 2 durchsetzendes elektrisches Feld 10 verursacht, wie dies in der Vergrößerung zu erkennen ist. Das elektrische Feld 10 bewirkt, dass sich die Piezoschicht 2 ausdehnt oder zusammenzieht, wodurch sich die Membranstruktur mit der Trägerschicht 1, den Elektrodenschichten 3 und 4 und der Piezoschicht 2 biegt. Wird eine Wechselspannung an der Spannungsquelle 5 angelegt, so kann die Membranstruktur in Schwingung versetzt werden.

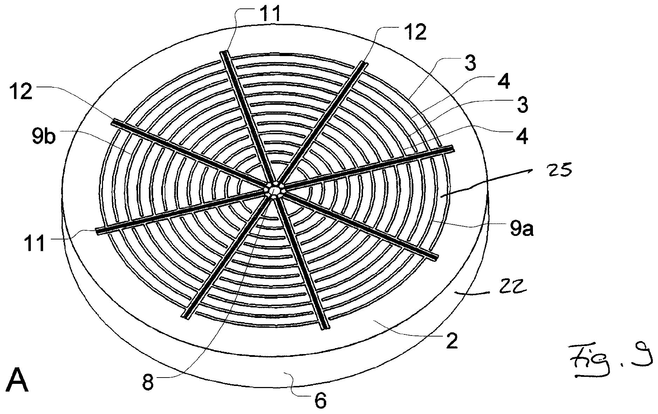

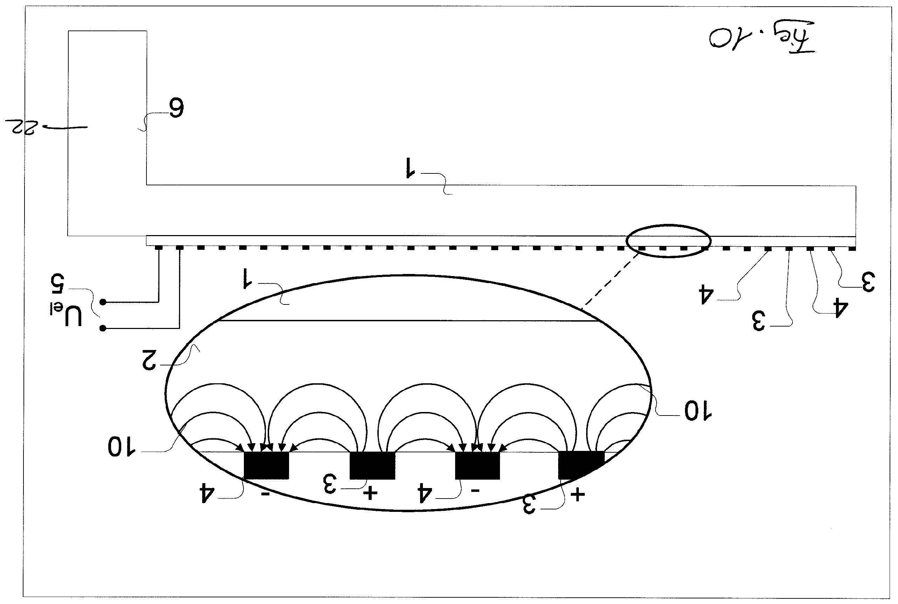

Figur 9 zeigt eine Aufsicht auf einen erfindungsgemäßen Schallwandler 22, in welchem die Elektroden wie in Figur 10 angeordnet sind. In der Ausführungsform der Figur 10 verlaufen die Elektroden auf der gezeigten Oberfläche. Es können auch vorzugsweise innerhalb der piezoelektrischen Schicht unterhalb der gezeigten Elektroden 3 und 4 weitere Elektroden 3 und 4 angeordnet sein. Die Elektroden 3 und 4 durchsetzen dann die piezoelektrische Schicht 2 in einer oder mehreren Ebenen.

Die in Figur 9 gezeigte Membran 25 ist wiederum kreisförmig, und die Elektroden sind als konzentrische Kreisabschnitte ausgeführt. Hierbei verlaufen eine Vielzahl von Elektroden 3 und 4 kreisförmig um den Mittelpunkt 8 der Membran, wobei sich die Polarität der Elektroden 3 und 4 vom Rand 6 in Richtung zum Mittelpunkt 8 abwechselt. Die in Figur 9 gezeigte Membran ist in acht Segmente 9a, 9b segmentiert, die an einem gemeinsamen Rand 6 fest angeordnet sind und gegeneinander mechanisch entkoppelt sind.

Die Vielzahl der Elektroden 3 und 4 sind im in Figur 9 gezeigten Beispiel durch Leiter 11 und 12 kontaktiert, die radial vom Rand 6 in Richtung der Mitte 8 verlaufen. Dabei sind stets Elektroden einer Polarität 3 von einem Leiter 11 und Elektroden der anderen Polarität 4 von einem anderen Leiter 12 kontaktiert. Es sind also stets eine Vielzahl von Elektroden 3 gleicher Polarität durch einen gemeinsamen Leiter 11 kontaktierbar.

Zu erkennen ist, dass die Elektroden der einen Polarität 4 und jene der anderen Polarität 3 kammförmig ineinandergreifen und an ihrem einen Ende durch einen gemeinsamen Leiter 11 bzw. 12 gemeinsam kontaktiert werden. Die Elektroden einer Polarität 4 verlaufen hierbei von ihrem gemeinsamen Leiter 12 in Richtung des Leiters 11 der anderen Polarität, enden jedoch, bevor sie diesen erreichen, so dass kein elektrischer Kontakt zwischen Elektroden 4 einer Polarität und einem Leiter 11 der anderen Polarität zustande kommt. Im Großteil des Bereiches zwischen zwei Leitern 11 und 12 unterschiedlicher Polarität verlaufen stets Elektroden 3 und 4 in radialer Richtung abwechselnd, so dass sich zwischen den Elektroden, wie vorstehend gezeigt, elektrische Felder ausbilden können, die die Piezoschicht durchsetzen und hierdurch eine Ausdehnung oder Zusammenziehung der Piezoschicht 2 bewirken können.

Figur 10 zeigt eine weitere Ausgestaltungsform des Schallwandlers 22. Die in Figur 10 gezeigte Ausführungsform kann auch ein Schnitt durch die in Figur 9 gezeigte Ausführungsform sein. Hierbei ist auf einer Trägerschicht 1 eine Piezoschicht 2 angeordnet, die im gezeigten Beispiel die Trägerschicht 1 unmittelbar berührt. Auf der der Trägerschicht 1 abgewandten Seite der Piezoschicht 2 sind nun bandförmige Elektroden 3, 4 mit abwechselnder Polarität nebeneinander und parallel zueinander angeordnet. Auf der der Trägerschicht 1 abgewandten Oberfläche der Piezoschicht 2 wechseln sich also im Schnittbild Elektroden der einen Polarität 3 mit den Elektroden der anderen Polarität 4 ab. Im Schnittbild in Figur 6 sind auch die bandförmigen Elektroden 3 und 4 im Schnitt gezeigt und haben hier einen wesentlichen rechteckigen Querschnitt. Die Elektroden 3 und 4 liegen äquidistant zueinander.

Zwischen je zwei benachbarten Elektroden 3 und 4 bildet sich nun ein elektrisches Feld 10 aus, das von einer der Elektroden 3 durch die Piezoschicht 2 zur benachbarten Elektrode entgegengesetzter Polarität 4 verläuft. Das elektrische Feld 10, das durch Anlegen einer Spannung an der Spannungsquelle 5 zwischen den Elektroden 3 und 4 entsteht, durchsetzt also die Piezoschicht 2. Diese ändert dadurch ihre Länge, so dass sich die Membranstruktur mit der Trägerschicht 1 und der Piezoschicht 2 nach oben oder unten biegt. Wie auch in den vorhergehenden Beispielen kann die Membranstruktur durch einen Rahmen 6 getragen werden und segmentiert oder zusammenhängend sein.

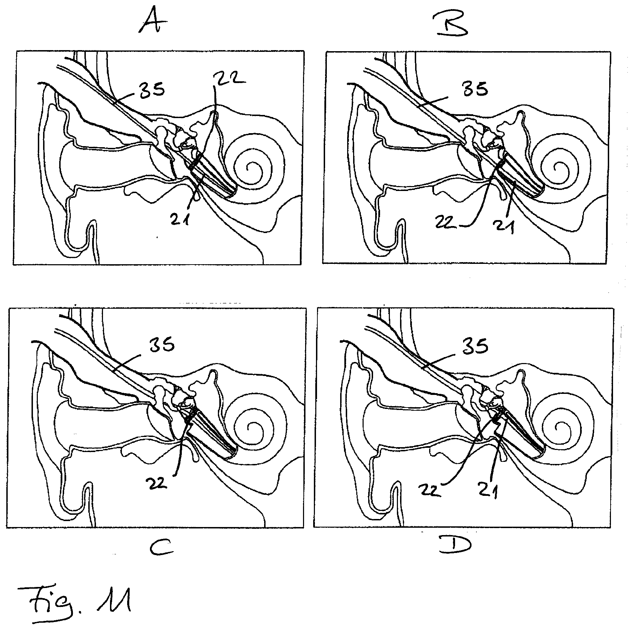

Figur 11 zeigt in den Teilfiguren A, B, C und D, wie das erfindungsgemäße elektroakustische Implantat im Gehör einer Person implantierbar ist. Dabei ist in den Teilfiguren 11A und 11B der Schallwandler 22 vor dem runden Fenster angeordnet, während er in den Teilfiguren 11C und 11D vor einem künstlich geschaffenen dritten Fenster angeordnet ist. In allen Teilfiguren erstreckt sich der Elektrodenträger 21 durch den Schallwandler 22 hindurch in die Schnecke.

In den Teilfiguren 11A und 11C durchtritt der Elektrodenträger 21 den Schallwandler 22 zentrisch, wie in den Figuren 1 und 2 gezeigt. In den Teilfiguren 11B und 11D erstreckt sich der Elektrodenträger 21 exzentrisch durch den Schallwandler 22, wie in den Figuren 3 und 4 gezeigt.

Der Elektrodenträger 21 kann eine Kontaktierung 35 nach außen aufweisen, so dass eine Energieversorgung für das Implantat und eine Stromversorgung außerhalb untergebracht sein können.

Die Leitung 35 kann durch eine chirurgisch geschaffene Öffnung im Mastoid (Teil des Schläfenbeins) verlaufen. Die Teilentfernung des Mastoids wird "Mastoidektomie" genannt und ist Teil des Standardprozedur zur Implantation eines Cl.

## Patentansprüche

1. Elektroakustisches Implantat, aufweisend
einen länglichen Elektrodenträger (21) mit einer Mehrzahl an Stimulationselektroden (23), der in eine Hörschnecke einführbar ist, sowie einen flächigen Schallwandler (22), der durch Anlegen einer Spannung zumindest bereichsweise zum Schwingen anregbar ist,
wobei der Schallwandler so ausgestaltet ist, dass er in, auf und/oder vor einem runden Fenster oder einem ovalen Fenster oder einem chirurgisch geschaffenen dritten Fenster eines Ohres und/oder in einer Rundfensternische eines Ohres, das entsprechende Fenster zumindest teilweise oder vollständig abdeckend, so anordenbar ist, dass Schwingungen des Schallwandlers (22) Schallschwingungen durch das entsprechende Fenster bewirken,
wobei der längliche Elektrodenträger (21) durch eine Fläche des flächigen Schallwandlers (22) verläuft, die sich, wenn der Schallwandler (22) vor dem entsprechenden Fenster angeordnet ist, zumindest zum Teil über das entsprechende Fenster erstreckt, wobei der flächige Schallwandler (22) eine Membranstruktur (25) als Teil seiner Fläche aufweist,
wobei die Membranstruktur (25) zumindest eine Trägerschicht (1) und zumindest eine auf der Trägerschicht (1) angeordnete, zumindest ein piezoelektrisches Material aufweisende, Piezoschicht (2) aufweist, so dass durch Anlegen der Spannung an die Piezoschicht (2) der Schallwandler zumindest bereichsweise zum Schwingen anregbar ist, wobei die Membranstruktur (29) in der Fläche durch zumindest eine, alle Schichten der Membranstruktur (25) durchtrennende, Schnittlinie in zumindest zwei oder mehr Segmente (9a, 9b, 9c) unterteilt ist, so dass die Membranstruktur (25) an der Schnittlinie mechanisch entkoppelt ist.

2. Elektroakustisches Implantat nach dem vorhergehenden Anspruch, wobei der Schallwandler (22) im Mittelohr so fixierbar ist, dass seine Schwingung auf eine Flüssigkeit in der Hörschnecke übertragbar ist.

3. Elektroakustisches Implantat nach einem der vorhergehenden Ansprüche,
wobei der längliche Elektrodenträger (21) durch eine Mitte der Fläche des flächigen Schallwandlers (22) verläuft.

4. Elektroakustisches Implantat nach einem der Ansprüche 1 oder 2, wobei der längliche Elektrodenträger (21) exzentrisch durch die Fläche des flächigen Schallwandlers (22) verläuft.

5. Elektroakustisches Implantat nach einem der Ansprüche 1 oder 2, wobei der längliche Elektrodenträger (21) durch eine Einkerbung (34) am Rand der Fläche des flächigen Schallwandlers (22) durch diesen verläuft.

6. Elektroakustisches Implantat nach einem der vorhergehenden Ansprüche,
wobei der längliche Elektrodenträger (21) eine Cochleaimplantat-Elektrode ist, deren Länge so ausgestaltet ist, dass mit der Cochleaimplantat-Elektrode im implantierten Zustand nur Frequenzen größer oder gleich 1000 Hz, vorzugsweise größer oder gleich 1300 Hz, besonders bevorzugt größer oder gleich 1500 Hz anregbar sind.

7. Elektroakustisches Implantat nach einem der vorhergehenden Ansprüche,
wobei der Schallwandler (22) gegenüber dem Elektrodenträger (21) in einer Längsrichtung des Elektrodenträgers (21) verschiebbar ist.

8. Elektroakustisches Implantat nach einem der vorhergehenden Ansprüche,
wobei der Schallwandler (22) gegenüber dem Elektrodenträger (21) verkippbar ist, wobei vorzugsweise der Schallwandler (22) und der Elektrodenträger (21) über ein Kugelgelenk (33) miteinander verbunden sind.

9. Elektroakustisches Implantat nach einem der vorhergehenden Ansprüche,
wobei der Elektrodenträger (21) durch eine Öffnung (30) in der Fläche des Schallwandlers (22) verläuft, deren Durchmesser größer ist als ein Durchmesser des Elektrodenträgers (21), so dass der Elektrodenträger (21) zumindest bereichsweise von einer Wand der Öffnung (30) beabstandet ist.

## Claims

1. An electro-acoustic implant, comprising
an elongate electrode carrier (21) having a plurality of stimulation electrodes (23) that can be introduced into a cochlea; and
a flat sound transducer (22) that is excitable to vibrate at least regionally by applying a voltage,
wherein the sound transducer is configured such that it is arrangeable in, on and/or in front of a round window or an oval window or a surgically created third window of an ear and/or in a round window niche of an ear, covering the corresponding window at least partially or completely, such that vibrations of the sound transducer (22) effect sound vibrations through the corresponding window;
wherein the elongate electrode carrier (21) extends through a plane of the flat sound transducer (22) that extends, when the sound transducer (22) is arranged in front of the corresponding window, at least partially over the corresponding window,
wherein the flat transducer (22) has a membrane structure (25) as a part of its plane; and
wherein the membrane structure (25) has at least one carrier layer (1) and at least one piezo layer (2) that is arranged on the carrier layer (1) and that comprises a piezoelectric material such that the sound transducer is excitable to vibrate at least regionally by applying the voltage to the piezo layer (2),
wherein the membrane structure (29) in the plane is divided by at least one cutting line separating all the layers of the membrane structure (25) into at least two or more segments (9a, 9b, 9c) such that the membrane structure (29) is mechanically decoupled at the cutting line.

2. An electro-acoustic implant in accordance with one of the preceding claims,
wherein the sound transducer (22) is fixable in the middle ear such that its vibration is transferable to a fluid in the cochlea.

3. An electro-acoustic implant in accordance with one of the preceding claims,
wherein the elongate electrode carrier (21) extends through a center of the plane of the flat sound transducer (22).

4. An electro-acoustic implant in accordance with one of the claims 1 or 2,
wherein the elongate electrode carrier (21) extends eccentrically through the plane of the flat sound transducer (22).

5. An electro-acoustic implant in accordance with one of the claims 1 or 2,
wherein the elongate electrode carrier (21) extends through the flat sound transducer (22) through a notch (34) at the edge of the plane thereof.

6. An electro-acoustic implant in accordance with one of the preceding claims,
wherein the elongate electrode carrier (22) is a cochlea implant electrode whose length is designed such that only frequencies larger than or equal to 1000 Hz, preferably larger than or equal to 1300 Hz, and particularly preferably larger than or equal to 1500 Hz, are excitable by the cochlea implant electrode in the implanted state.

7. An electro-acoustic implant in accordance with one of the preceding claims,
wherein the sound transducer (22) is displaceable with respect to the electrode carrier (21) in a longitudinal direction of the electrode carrier (21).

8. An electro-acoustic implant in accordance with one of the preceding claims,
wherein the sound transducer (22) is tiltable with respect to the electrode carrier (21), with the sound transducer (22) and the electrode carrier (21) preferably being connected to one another via a ball joint (35).

9. An electro-acoustic implant in accordance with one of the preceding claims,
wherein the electrode carrier (21) extends through an opening (30) in the plane of the sound transducer (22) whose diameter is larger than a diameter of the electrode carrier (21) such that the electrode carrier (21) is at least regionally spaced apart from a wall of the opening (30).

## Revendications

1. Implant électroacoustique, comportant
un support d'électrodes (21) de forme allongée avec une multiplicité d'électrodes de stimulation (23) qui peut être introduit dans une cochlée, ainsi qu'un transducteur acoustique (22) plan qui peut être excité en vibration au moins par endroits par l'application d'une tension,
dans lequel le transducteur acoustique est constitué de telle sorte qu'il peut être disposé dans, sur et/ou devant une fenêtre ronde, ou une fenêtre ovale ou une troisième fenêtre créée chirurgicalement d'une oreille et/ou dans une niche de fenêtre ronde d'une oreille, en recouvrant la fenêtre correspondante au moins partiellement ou totalement de sorte que des vibrations du transducteur acoustique (22) provoquent des vibrations acoustiques à travers la fenêtre correspondante,
dans lequel le support d'électrodes (21) de forme allongée s'étend à travers une surface du transducteur acoustique (22) plan qui, quand le transducteur acoustique (22) est disposé devant la fenêtre correspondante, s'étend au moins partiellement sur la fenêtre correspondante,
dans lequel le transducteur acoustique (22) plan comporte une structure membranaire (25) en tant que partie de sa surface,
dans lequel la structure membranaire (25) comporte au moins une couche de support (1) et au moins une couche piézoélectrique (2) disposée sur la couche de support (1), comportant au moins un matériau piézoélectrique de telle sorte que, par l'application de la tension sur la couche piézoélectrique (2), le transducteur acoustique peut être excité en vibration au moins par endroits,
dans lequel la structure membranaire (29) dans la surface est divisée en au moins deux segments (9a, 9b, 9c) ou plus par au moins une ligne de coupe coupant toutes les couches de la structure membranaire (25) de telle sorte que la structure membranaire (25) est découplée mécaniquement au niveau de la ligne de coupe.

2. Implant électroacoustique selon la revendication précédente,
dans lequel le transducteur acoustique (22) peut être fixé dans l'oreille moyenne de telle sorte que sa vibration peut être transmise à un liquide dans la cochlée.

3. Implant électroacoustique selon l'une des revendications précédentes,
dans lequel le support d'électrodes (21) de forme allongée s'étend à travers un milieu de la surface du transducteur acoustique (22) plan.

4. Implant électroacoustique selon l'une des revendications 1 ou 2,
dans lequel le support d'électrodes (21) de forme allongée s'étend de façon excentrée à travers la surface du transducteur acoustique (22) plan.

5. Implant électroacoustique selon l'une des revendications 1 ou 2,
dans lequel le support d'électrodes (21) de forme allongée s'étend à travers une encoche (34) sur le bord de la surface du transducteur acoustique (22) plan à travers celui-ci.

6. Implant électroacoustique selon l'une des revendications précédentes,
dans lequel le support d'électrodes (21) de forme allongée est une électrode d'implant de cochlée dont la longueur est constituée de telle sorte que, quand l'électrode d'implant de cochlée est dans l'état implanté, seules des fréquences supérieures ou égales à 1 000 Hz, de préférence supérieures ou égales à 1 300 Hz, particulièrement préférablement supérieures ou égales à 1 500 Hz peuvent être excitées.

7. Implant électroacoustique selon l'une des revendications précédentes,
dans lequel le transducteur acoustique (22) peut être déplacé par rapport au support d'électrodes (21) dans une direction longitudinale du support d'électrodes (21).

8. Implant électroacoustique selon l'une des revendications précédentes,
dans lequel le transducteur acoustique (22) peut être basculé par rapport au support d'électrodes (21), dans lequel de préférence le transducteur acoustique (22) et le support d'électrodes (21) sont raccordés l'un à l'autre par le biais d'une articulation à rotule (33).

9. Implant électroacoustique selon l'une des revendications précédentes,
dans lequel le support d'électrodes (21) s'étend à travers une ouverture (30) dans la surface du transducteur acoustique (22) dont le diamètre est supérieur à un diamètre du support d'électrodes (21), de sorte que le support d'électrodes (21) est, au moins par endroits, espacé d'une paroi de l'ouverture (30).
